# EUROPÄISCHE PATENTSCHRIFT

(11) **EP 3 013 392 B1**
(45) Veröffentlichungstag und Bekanntmachung des Hinweises auf die Patenterteilung: **10.06.2020**
(21) Anmeldenummer: 14734374.3
(22) Anmeldetag: 05.06.2014
(51) Int. Cl.: A61M 5/20

(54) **AKTIVATOR FÜR EINEN AUTOINJEKTOR**
ACTIVATOR FOR AN AUTOMATIC INJECTION DEVICE
ACTIVATEUR POUR UNE AUTO-INJECTEUR

(30) Priorität: 24.06.2013 AT 5142013
(43) Veröffentlichungstag der Anmeldung: 04.05.2016
(73) Patentinhaber: Pharma Consult Ges.m.b.H., 1210 Wien (AT)
(72) Erfinder: SCHWIRTZ, Andreas, A-1090 Wien (AT); CSENAR, Markus, A-1050 Wien (AT)
(74) Vertreter: Walker, Ross Thomson
(86) Internationale Anmeldenummer: PCT/AT2014/000117
(87) Internationale Veröffentlichungsnummer: WO 2014/205463

(56) Entgegenhaltungen:
- WO-A1-2009/147026
- WO-A1-2012/073035
- WO-A2-2009/022132
- WO-A2-2012/049468
- US-A- 5 599 309
- US-A1- 2011 125 100

## Beschreibung

Die Erfindung betrifft eine Aktivatoreinheit für einen Autoinjektor mit einem im wesentlichen zylindrischen Gehäuse, in welchem ein axialverschieblicher Druckbolzen geführt ist, welcher gegen eine Federeinheit einschiebbar und in der eingeschobenen Lage mittels Rastfortsätzen eines mit dem Druckbolzen verbundenen Halterungselementes verriegelbar ist. Die Erfindung betrifft ebenso einen Autoinjektor mit der Aktivatoreinheit.

Aktivatoren der eingangs genannten Art werden als Bestandteil von medizinischen Injektionsspritzen verwendet, die einfach, automatisch und meistens einmalig zu bedienen sind. Eine solche als Autoinjektor bezeichnete Spritze wird dabei an ihrem distalen Ende auf einen Bereich des Interesses des Körpers eines Menschen aufgelegt und anschließend häufig im proximalen Bereich des Autoinjektors durch einen einfachen Handgriff ausgelöst. Die Ausdrücke "proximal" und "distal" beziehen sich auf die Perspektive des Anwenders eines Autoinjektors.

Die WO 2005/021070 A1 zeigt eine Vorrichtung zum automatischen Injizieren von Injektionsflüssigkeiten. Ein Teil dieser Vorrichtung ist auch ein Aktivator in einem zylindrischen Gehäuse, in welchem eine Kolbenstange durch eine Feder schiebbar ist. Wird die Feder ausgelöst und die Kolbenstange anschließend zum distalen Ende des Aktivators hin hinausgeschoben, wird eine Karpule mit einer Injektionsnadel verbunden, die Nadel aus der Vorrichtung zum automatischen Injizieren herausgeschoben und die Kolbenstange in die Karpule hineingedrückt, so daß ein flüssiges Medikament durch die Nadel injiziert wird. Optional kann die Nadel bereits mit der Karpule verbunden sein. Nachteilig bei dieser Lehre ist, daß der Kraftspeicher des Aktivators aus einer Spiralfeder besteht. Dies führt dazu, daß die Kraft, die dazu erforderlich ist, die Flüssigkeit zu injizieren, erst am Ende des automatischen Injektionsablaufes und somit durch die Spiralfeder im nahezu entspannten Zustand vermittelt werden kann. Die zur Verfügung stehende Kraft reicht zwar aus, um ein flüssiges Medikament durch die Kanüle zu pressen. Sie reicht aber nicht mehr dafür aus, ein dickflüssiges Medikament durch die Kanüle und/oder das Medikament durch eine Kanüle mit einem geringeren Durchmesser zu pressen.

Die Erfindung zielt darauf ab, einen Aktivator wie eingangs angeführt zu schaffen, welcher eine höhere Kraft ausübt, wenn das Medikament injiziert werden soll. Weiterhin soll der Aktivator nicht wesentlich aufwendiger und teurer als Aktivatoren des Standes der Technik herzustellen sein; er soll ebenso im Vergleich zu den Aktivatoren des Standes der Technik einfach und sicher zu bedienen sein sowie keine größeren äußeren Abmessungen aufweisen. Eine weitere Aufgabe besteht darin, die Bedienung eines Autoinjektors für den Patienten angenehmer zu machen.

Der erfindungsgemäße Aktivator erreicht dies dadurch, daß die Federeinheit eine erste Spiralfeder sowie mindestens eine weitere zweite Spiralfeder mit einem größeren Durchmesser als die erste Spiralfeder umfaßt, wobei die zweite Spiralfeder koaxial zu der ersten Spiralfeder angeordnet ist, wobei die Spiralfedern so angeordnet sind, dass sie sich gegenseitig führen. Eine Kolbenstange, die sich vom Kolben bis zum proximalen Ende des Aktivators erstreckt, ist nicht mehr vorgesehen.

Eine bevorzugte Ausführungsform der Aktivatoreinheit zeichnet sich dadurch aus, daß die erste Spiralfeder und die zweite Spiralfeder im wesentlichen die gleiche Länge aufweisen.

In einer Ausführungsform der Erfindung sind die erste Spiralfeder und die zweite Spiralfeder als Teile einer einstückigen Doppelfeder ausgeführt.

Zur weiteren Ausgestaltung der Erfindung ist die zweite Spiralfeder mit einem durch die erste Spiralfeder verschieblichen Organ gehaltert und verriegelt, wobei die Verriegelung der zweiten Spiralfeder an einem äußeren Entspannungspunkt der ersten Spiralfeder entriegelbar ist.

In einer anderen Ausführungsform der Erfindung ist die erste Spiralfeder mit einem durch die zweite Spiralfeder verschieblichen Organ gehaltert und verriegelt, wobei die Verriegelung der ersten Spiralfeder in einem äußeren Entspannungspunkt der zweiten Spiralfeder entriegelbar ist.

Bevorzugt ist in einer Ausgestaltung der Erfindung, daß die Aktivatoreinheit eine in axialer Richtung verschiebbare Aktivatorhülse aufweist, durch welche die Rastfortsätze derart verschiebbar sind, daß die Verriegelung lösbar und der Druckbolzen durch die Federeinheit bewegbar wird.

Weiterhin bevorzugt wird in Ausgestaltung der erfindungsgemäßen Vorrichtung, daß die Aktivatoreinheit eine entfernbare Sicherungskappe aufweist, welche ein Organ aufweist, das die Rastfortsätze fixiert.

Die Erfindung weist die Aktivatoreinheit an ihrem distalen Ende ein Gewinde oder einen Bajonettverschluß auf, durch welches/welchen sie mit einer Injektoreinheit verbindbar ist.

Bevorzugt ist in einer Ausgestaltung der Erfindung, daß der Druckbolzen an seiner distalen Stirnseite mechanische Mittel zum Brechen einer Versiegelung aufweist.

Die Erfindung betrifft auch einen Autoinjektor, der eine erfindungsgemäße Aktivatoreinheit aufweist.

Die Erfindung wird nachstehend anhand eines in den Zeichnungen dargestellten Ausführungsbeispieles näher erläutert. Es zeigen: Fig. 1 einen Querschnitt durch einen Autoinjektor, Fig. 2 eine Explosionsansicht der Aktivatoreinheit, und Fig. 3 bis 6 schematische Querschnitte durch einen Autoinjektor.

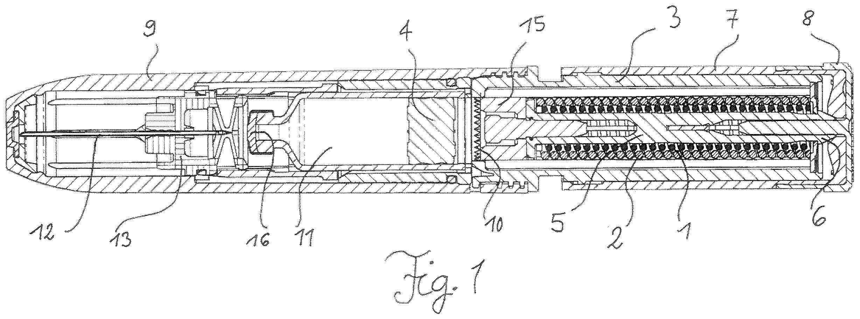

Gemäß Fig. 1 weist ein Autoinjektor an seinem distalen Ende eine Injektionseinheit 9 auf. In ihr befinden sich eine Karpule 11, die das Medikament in sich trägt, sowie die Nadel 12. Die Karpule 11 ist an ihrem distalen Ende mit einer Karpulenversiegelung 16 verschlossen. An ihrem proximalen Ende weist die Karpule 11 einen Druckbolzen 4 auf, der in die Karpule hinein verschieblich ist. Die Nadel 12 ist über einen Nadelhalter 13 gehaltert. Am proximalen Ende des Autoinjektors befindet sich die Aktivatoreinheit. Sie ist mit der Injektionseinheit 9 verschraubt oder anderweitig verbunden und gemäß Fig. 1 im gesicherten Zustand dargestellt, d.h. eine erste Spiralfeder 1 und eine zweite Spiralfeder 2 befinden sich im gespannten Zustand zwischen einem Kolben 15 am distalen Ende und einer Verriegelung am proximalen Ende. Um die Verriegelung aufrechtzuerhalten, hält ein Halterungselement 5 den Kolben 15 über Rastfortsätze 6 fest. Die jeweilige Verschiebung der Rastfortsätze 6 entlang der Richtung der Längsachse der Aktivatoreinheit wird durch eine Sicherungskappe 8 verhindert, die zentral einen Stift aufweist, welcher die Bewegungsfreiheit der Rastfortstäze 6 einschränkt. Weiterhin befindet sich eine Aktivatorhülse 7 umfänglich am Gehäuse 3 der Aktivatoreinheit und ist zum distalen Ende der Aktivatoreinheit hin und zum Autoinjektor insgesamt verschieblich. Im bisherigen Stand der Technik war es nicht möglich, sowohl eine erste Spiralfeder 1 als auch eine zweite Spiralfeder 2 in der Aktivatoreinheit unterzubringen. Der Grund dafür lag darin, daß der Autoinjektor insgesamt und die Aktivatoreinheit ein handliches Instrument bilden müssen und daher der Platz in der Aktivatoreinheit stark begrenzt ist. Da Autoinjektoren herkömmlichen Injektionsspritzen bislang entlehnt waren, hat man eine Spiralfeder im Inneren der hohl ausgeführten Kolbenstange oder im Hohlraum innerhalb der Spiralfeder angeordnet. Da die Kolbenstange ihrerseits Raum beansprucht, war daher nur Platz für eine Feder und somit eine natürliche Obergrenze für den verfügbaren Druck auf den Kolben gesetzt. Das Weglassen der Kolbenstange wurde bisher nicht in Betracht gezogen, weil dadurch die mechanischen Elemente, insbesondere im Bewegungsablauf, unkontrollierbare Freiheitsgrade erhalten hätten. Gemäß der Erfindung wird aber gleichzeitig die lange Kolbenstange entfernt und der frei gewordene Raum durch eine zweite Spiralfeder eingenommen. Der dadurch auftretende überraschende Effekt liegt darin, daß sich die Federn sowohl im gespannten verriegelten Zustand als auch während des Injektionsvorganges gegenseitig führen und eine Bewegung nur in der Richtung der Entspannung, d.h. in Richtung des distalen Endes der Aktivatoreinheit, möglich ist. Auf diesem Wege ist es möglich, die Federkraft auf Druckbolzen 4 und Kolben 15 wesentlich zu erhöhen, die Baugröße der Aktivatoreinheit in ihren Außenmaßen jedoch nicht wesentlich zu verändern.

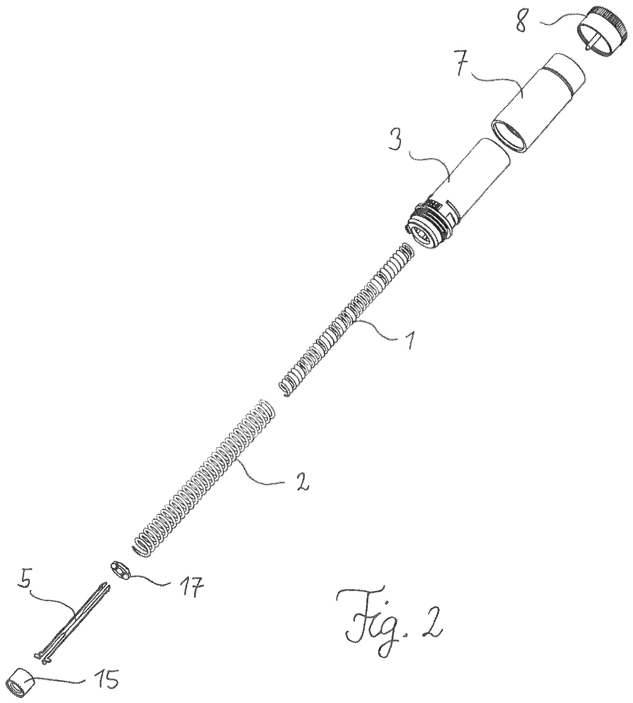

In Fig. 2 ist dargestellt, wie der bisherige Kolben, bestehend aus distalem Kolbenende und Kolbenstange, nur noch durch den in Fig. 2 dargestellten einfachen Kolben 15 ersetzt ist. Das Halterungselement 5 wird durch die erste Spiralfeder 1 und die zweite Spiralfeder 2 durchgesteckt und an einer proximalen Öffnung im Gehäuse 3 eingerastet. Beide Federn sitzen auf dem Kolben 15 über einen Federadapter 17 auf. Auf das Gehäuse 3 ist dann nur noch die Aktivatorhülse 7 und die Sicherungskappe 8 gesteckt. Die getrennt von der Injektionseinheit 9 herstellbare und lagerbare Aktivatoreinheit muß nicht steril sein. Erst im Augenblick der Injektion drückt der Kolben 15 auf den Druckbolzen 4 der Injektionseinheit 9; davor ist der Kolben 15 in der Aktivatoreinheit verriegelt und der Druckbolzen 5 innerhalb der Karpule 11 in der sterilen Injektionseinheit 9 gelagert. Zur Verbesserung der Sterilitätsanforderungen an die Injektionseinheit 9 ist sie an ihrem proximalen Ende durch eine dünne Folie eines geeigneten Materials versiegelt. Auch während die Aktivatoreinheit mit der Injektionseinheit 9 verbunden ist, bleibt die Versiegelung aufrecht, weil Druckbolzen 4 und Kolben 15 voneinander beabstandet sind. Vorteilhaft für den reibungslosen Ablauf ist es dann jedoch, wenn der Kolben 15 ein mechanisches Mittel zum Brechen der Versiegelung aufweist. Dieses Mittel kann beispielsweise darin bestehen, daß der Kolben 15 an seiner distalen Stirnseite einen Kranz oder eine andere geometrische Anordnung von Zähnen aufweist, die die folienartige Versiegelung am Beginn des Injektionsvorganges aufschlitzen.

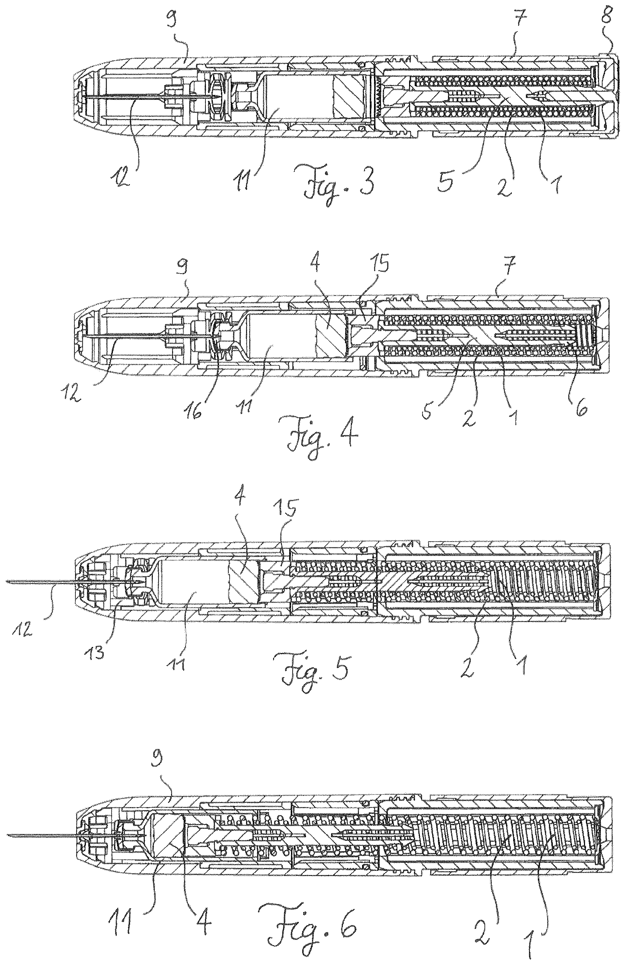

Fig. 3 zeigt einen Autoinjektor mit der erfindungsgemäßen Aktivatoreinheit und der mit ihr verbundenen Injektionseinheit 9 in Ausgangsposition. Die Karpule 11 ist versiegelt und noch nicht mit der Nadel 12 verbunden. Die erste Spiralfeder 1 und die zweite Spiralfeder 2 sind gespannt und durch das Halterungselement 5 verriegelt, welches durch die Sicherungskappe 8 fixiert ist.

Die Fig. 4, 5 und 6 zeigen den Ablauf des Injizierens anhand dreier Zustände des Autoinjektors. In Fig. 4 ist die Schutzkappe 8 bereits entfernt und die Aktivatorhülse 7 in distaler Richtung verschoben. Dabei ist der Autoinjektor beispielsweise an seinem distalen Ende auf einem Körperteil eines Patienten aufgesetzt (nicht gezeigt). Durch das Verschieben der Aktivatorhülse 7 wurden die Rastfortsätze 6 des Halterungselementes 5 zusammengedrückt, was die Verriegelung aufhebt. Die erste Spiralfeder 1 sowie die zweite Spiralfeder 2 beginnen sich zu entspannen. Dabei wird Druck auf den Kolben 15 ausgeübt, der sich in distaler Richtung anfängt zu bewegen. Dies führt zunächst dazu, daß er auf dem Druckbolzen 4 aufsetzt und auch diesen in Bewegung bringt. Die Injektionseinheit 9 ist insgesamt nun so eingestellt, daß sich der Druckbolzen 4 nicht in der Karpule 11 bewegt, sondern die Karpule 11 mit sich führt und gegen die Nadel 12 drückt. Dieser sticht nun durch die Karpulenversiegelung 16 und verbindet sich so mit der Karpule 11 selbst.

Nach dem vollständigen Verbinden der Nadel 12 mit der Karpule 11 werden nun durch den Federdruck sowohl der ersten Spiralfeder 1 als auch der zweiten Spiralfeder 2 der Druckbolzen 4, die Karpule 11 mitsamt dem Medikament und der Nadel 12 in distaler Richtung weiterbewegt. Eine allfällige Versiegelung der Injektionseinheit 9 an ihrem distalen Ende wird durch die Nadel 12 durchstochen, und die Nadel 12 dringt in den Körperteil des Patienten ein. Da nun der Nadelhalter 13 und die Karpule 11 an ihrem distalen Endpunkt gelangen und in der Injektionseinheit 9 zum Stillstand gelangen, führt der weiterhin bestehende Druck der ersten Spiralfeder 1 und der zweiten Spiralfeder 2 über den Kolben 15 auf den Druckbolzen 4 dazu, daß nun das Medikament über die Nadel 12 ausgespritzt wird. In diesem letzten Schritt entfaltet sich die hohe Druckkraft zweier parallel wirkender Federn.

In Fig. 6 ist die Endposition des Autoinjektors mit der Injektionseinheit 9 und der erfindungsgemäßen Aktivatoreinheit dargestellt. Sowohl die erste Spiralfeder 1 als auch die zweite Spiralfeder 2 haben den Druckbolzen 4 bis an das distale Ende der Karpule 11 bewegt. Das Medikament ist nun vollständig ausgespritzt.

Bei der in den Figuren gezeigten Ausführungsform des Autoinjektors mit der erfindungsgemäßen Aktivatoreinheit arbeiten beide Federn gleichzeitig. Es ist ebenso durch im Stand der Technik bekannte Teleskophülsen möglich, einen seriellen Bewegungsablauf der ersten Spiralfeder 1 und der zweiten Spiralfeder 2 zu erreichen. Bei einer solchen Ausführungsform schiebt die erste Spiralfeder 1 die Karpule 11 und die Nadel 12 sowie die zweite Spiralfeder 2 in distaler Richtung. Während dieses Vorganges ist die zweite Spiralfeder 2 mit einem Organ verriegelt und in diesem gehaltert. Dieses Organ kann eine entsprechende Teleskophülse sein. Die Entriegelung der zweiten Spiralfeder 2 erfolgt kurz vor oder genau dann, wenn die Karpule 11 zusammen mit der Nadel 12 ihren maximalen distalen Punkt erreicht haben. In diesem Augenblick bringt die zweite Spiralfeder 2 die Kraft auf, um das Medikament auszustoßen. Vorteilhaft bei dieser Ausführungsform ist, daß eine abermals erhöhte Kraft für das Ausstoßen des Medikamentes zur Verfügung steht. Es ist auch umgekehrt möglich, die erste Feder 1 verriegelt durch die bereits zuerst ausgelöste zweite Feder 2 zum distalen Ende zu verschieben und die erste Feder 1 erst auszulösen, wenn Karpule 11 und Nadel 12 am distalen Ende des Autoinjektors angekommen sind oder kurz davor sind.

Die gestaffelte Entriegelung ist besonders bei hochviskosen Wirkstoffen erforderlich, was bedeutet, daß eine erhöhte Federkraft nicht so sehr am Anfang des Bewegungsablaufes des Autoinjektors erforderlich ist, wo nur die Karpule 11 mit der Nadel 12 zu verbinden ist und die Nadel 12 in den Körperteil des Patienten einzustechen sind, sondern vielmehr am Ende des Bewegungsablaufes, wenn es nur noch darum geht, den Druckbolzen 4 durch die Karpule 11 zu schieben.

Es stellt sich ferner der Vorteil ein, daß die Rückschlagkraft, die bei Auslösen des Aktivators auftritt und die von Patienten während der Anwendung als unangenehm empfunden wird, bei dem erfindungsgemäßen Aktivator geringer ausfällt als bei den Aktivatoren des Standes der Technik.

Verschiedene Abwandlungen der genannten Ausführungsformen sind denkbar. So kann zum Beispiel die erste Spiralfeder 1 mit der zweiten Spiralfeder 2 verbunden sein und eine einstückige Doppelfeder bilden. Dies kann sich vorteilhaft im Herstellungsverfahren der Spiralfeder selbst und beim Zusammenbau der Aktivatoreinheit erweisen. Der Vorteil der erhöhten Kraft, die durch zwei Spiralfedern auf den Kolben 15 wirken, bleibt dabei weiterhin erhalten.

## Patentansprüche

1. Aktivatoreinheit für einen Autoinjektor mit einem im Wesentlichen zylindrischen Gehäuse (3), in welchem ein axial verschiebarer Kolben (15) geführt ist, welcher gegen eine Federeinheit einschiebbar und in der eingeschobenen Lage mittels Rastfortsätzen (6) eines mit dem Kolben (15) verbundenen Halterungselementes (5) verriegelbar ist,
wobei die Aktivatoreinheit weiter an ihrem distalen Ende ein Gewinde oder einen Bajonettverschluss aufweist, durch welches/welchen sie mit einer Injektoreinheit (9) verbindbar ist, um einen Autoinjektor zu bilden;
wobei die Federeinheit eine erste Spiralfeder (1) sowie mindestens eine weitere zweite Spiralfeder (2) mit einem größeren Durchmesser als die erste Spiralfeder (1) umfasst, und wobei die zweite Spiralfeder (2) koaxial zu der ersten Spiralfeder (1) angeordnet ist, **dadurch gekennzeichnet, dass** die Spiralfedern so angeordnet sind,
dass sie sich gegenseitig führen.

2. Aktivatoreinheit nach Anspruch 1, **dadurch gekennzeichnet, dass** die erste Spiralfeder (1) und die zweite Spiralfeder (2) im Wesentlichen die gleiche Länge aufweisen.

3. Aktivatoreinheit nach Anspruch 1 oder 2, **dadurch gekennzeichnet, dass** die erste Spiralfeder (1) und die zweite Spiralfeder (2) als Teile einer einstückigen Doppelfeder ausgeführt sind.

4. Aktivatoreinheit nach Anspruch 1, **dadurch gekennzeichnet, dass** die zweite Spiralfeder (2) mit einem durch die erste Spiralfeder (1) verschieblichen Organ gehaltert und verriegelt ist, wobei die Verriegelung der zweiten Spiralfeder (2) in einem äußeren Entspannungspunkt der ersten Spiralfeder (1) entriegelbar ist.

5. Aktivatoreinheit nach Anspruch 1, **dadurch gekennzeichnet, dass** die erste Spiralfeder (1) mit einem durch die zweite Spiralfeder (1) verschieblichen Organ gehaltert und verriegelt ist, wobei die Verriegelung der ersten Spiralfeder (1) in einem äußeren Entspannungspunkt der zweiten Spiralfeder (2) entriegelbar ist.

6. Aktivatoreinheit nach einem der Ansprüche 1 bis 5 **dadurch gekennzeichnet, dass** die Aktivatoreinheit eine in axialer Richtung verschiebbare Aktivatorhülse (7) aufweist, durch welche die Rastfortsätze (6) derart verschiebbar sind, dass die Verriegelung losbar und der Kolben (15) durch die Federeinheit bewegbar wird.

7. Aktivatoreinheit nach Anspruch 6, **dadurch gekennzeichnet, dass** die Aktivatoreinheit eine entfernbare Sicherungskappe (8) aufweist, welche ein Organ aufweist, das die Rastfortsätze fixiert.

8. Aktivatoreinheit nach Anspruch 1, **dadurch gekennzeichnet, dass** der der Kolben (15) an seiner distalen Stirnseite mechanische Mittel zum Brechen einer Versiegelung aufweist.

9. Autoinjektor mit einer Aktivatoreinheit gemäß der Ansprüche 1 bis 8.

## Claims

1. Activator unit for an autoinjector with an essentially cylindrical housing (3) wherein an axially displaceable piston (15) is guided that can be inserted counter to the spring unit and locked in the inserted position by means of detent projections (6) of a retaining element (5) connected to the piston (15), wherein the activator unit further comprises at its distal end t its distal end a thread or a bayonet lock by which it can be connected to an injector unit (9) to form an autoinjector; wherein the spring unit comprises a first coil spring (1) and at least one further second coil spring (2) having a larger diameter than the first coil spring (1), and wherein the second coil spring (2) is coaxial to the first coil spring (1), **characterized in that** the coil springs are configured to guide one another.

2. Activator unit according to claim 1, **characterized in that** the first coil spring (1) and the second coil spring (2) have essentially the same length.

3. Activator unit according to claim 1 or 2, **characterized in that** the first coil spring (1) and the second coil spring (2) are designed as parts of a one-piece double spring.

4. Activator unit according to claim 1, **characterized in that** the second spiral spring (2) is held by and locked with an element that can be displaced by the first spiral spring (1), wherein the locking of the second spiral spring (2) can be unlocked at a distal release point of the first spiral spring (1).

5. Activator unit according to claim 1, **characterized in that** the first spiral spring (1) is held by and locked with an element that can be displaced by the second spiral spring (2), wherein the locking of the first spiral spring (1) can be unlocked at a distal release point of the second spiral spring (2).

6. Activator unit according to any one of claims 1 to 5, **characterized in that** the activator unit has an activator sleeve (7) that can be displaced in the axial direction and by which the detent projections (6) can be displaced in such a way that the lock can be released and the piston (15) becomes movable by the spring unit.

7. Activator unit according to claim 6, **characterized in that** the activator unit has a removable safety cap (8) which includes an element that fixes the detent projections in place.

8. Activator unit according to claim 1, **characterized in that** the piston (15) has at its distal end mechanical means to break a seal.

9. Autoinjector with an activator unit according to claims 1 to 8.

## Revendications

1. Ensemble activateur pour un auto-injecteur, avec un boîtier (3) sensiblement cylindrique, dans lequel est guidé un piston déplaçable axialement (15), qui peut être poussé vers l'intérieur contre un ensemble à ressorts, et qui, dans la position enfoncée, peut être verrouillé au moyen de saillies de blocage (6) d'un élément de maintien (5) qui est relié au piston (15),
l'ensemble activateur comprenant en sus à son extrémité distale un filetage ou un verrou à baïonnette, au moyen desquels il peut être relié à un ensemble injecteur (9), afin de former un auto-injecteur ;
l'ensemble à ressorts comprenant un premier ressort hélicoïdal (1), ainsi qu'au moins un deuxième ressort hélicoïdal (2) supplémentaire de diamètre supérieur à celui du premier ressort hélicoïdal (1), et
le deuxième ressort hélicoïdal (2) étant disposé coaxialement au premier ressort hélicoïdal (1), **caractérisé en ce que** les ressorts hélicoïdaux sont disposés de telle manière qu'ils se guident l'un l'autre.

2. Ensemble activateur selon la revendication 1, **caractérisé en ce que** le premier ressort hélicoïdal (1) et le deuxième ressort hélicoïdal (2) présentent sensiblement la même longueur.

3. Ensemble activateur selon la revendication 1 ou 2, **caractérisé en ce que** le premier ressort hélicoïdal (1) et le deuxième ressort hélicoïdal (2) sont configurés comme des parties d'un ressort monobloc double.

4. Ensemble activateur selon la revendication 1, **caractérisé en ce que** le deuxième ressort hélicoïdal (2) est maintenu et verrouillé par un organe qui peut être déplacé par le premier ressort hélicoïdal (1), le verrouillage du deuxième ressort hélicoïdal (2) pouvant être libéré en un point de détente externe du premier ressort hélicoïdal (1).

5. Ensemble activateur selon la revendication 1, **caractérisé en ce que** le premier ressort hélicoïdal (1) est maintenu et verrouillé par un organe qui peut être déplacé par le deuxième ressort hélicoïdal (1), le verrouillage du premier ressort hélicoïdal (1) pouvant être libéré en un point de détente externe du deuxième ressort hélicoïdal (2).

6. Ensemble activateur selon une quelconque des revendications 1 à 5, **caractérisé en ce que** l'ensemble activateur comprend un manchon d'activateur (7) qui peut être déplacé en direction axiale, au moyen duquel les saillies de blocage (6) peuvent être déplacées de manière à ce que l'ensemble de verrouillage puisse être libéré et le piston (15) puisse être déplacé par l'ensemble à ressorts.

7. Ensemble activateur selon la revendication 6, **caractérisé en ce que** l'ensemble activateur comprend un capuchon de fixation amovible (8), qui comprend un organe qui retient en place les saillies de blocage.

8. Ensemble activateur selon la revendication 1, **caractérisé en ce que** le piston (15) comprend sur son côté de face distale un moyen mécanique pour briser un joint.

9. Auto-injecteur avec un ensemble activateur selon les revendications 1 à 8.
